# NEW EUROPEAN PATENT SPECIFICATION

(11) **EP 0 547 497 B2**
(45) Date of publication and mention of the opposition decision: **05.07.2000**
(45) Mention of the grant of the patent: 26.03.1997
(21) Application number: 92121010.0
(22) Date of filing: 09.12.1992
(51) Int. Cl.: A61F 13/15

(54) **Disposable three dimensional garment and method of making same**
Wegwerfkleidungsstück und Verfahren zum Herstellen
Vêtement jetable et procédé de fabrication

(30) Priority: 18.12.1991 US 809993
(43) Date of publication of application: 23.06.1993
(60) Divisional application: 96113342.8
(73) Proprietor: KIMBERLY-CLARK WORLDWIDE, INC., Neenah, Wisconsin 54956 (US)
(72) Inventor: Van Gompel, Paul Theodore, Hortonville, WI 54944 (US); Suprise, Jody Dorothy, Neenah, WI 54956 (US); Schleinz, Robert Joseph, Appleton, WI 54915 (US); Ziegler, Diane Mildred, Appleton, WI 54914 (US); Popp, Robert Lee, Hortonville, WI 54944 (US); Poklasny, June Marjorie, Oshkosh, WI 54901 (US)
(74) Representative: Diehl, Hermann O. Th., Dr.

(56) References cited:
- EP-A- 0 323 634
- EP-A- 0 456 885
- WO-A-90/14949
- GB-A- 2 242 610
- GB-A- 2 244 422
- US-A- 4 701 171
- US-A- 4 940 464

## Description

This invention pertains to absorbent garments, and more particularly to a disposable three-dimensional garment and a method of making same.
When a child reaches an age in the range of about 15 to 30 months, the parent or parents generally desire to start toilet training the child so he or she can become independent of the parent. The training pant is intended for use when the child has reached an age at which he or she is ready to graduate to three-dimensional garments as a replacement for two-dimensional diapers previously used. Thus, a suitable training pant has closed sides and permits a child to raise and lower it easily along the legs without requiring the aid of a parent. At the same time, a training pant must provide features of liquid and solid absorbency and the prevention of waste leakage.

One example of a training pant is the child's training pant in U.S. Pat. No. 4,646,362, assigned to the assignee of the present invention. This patent describes a training pant having a two-ply outer cover, a body side liner, and an absorbent therebetween. The training pant has an elasticized waist opening and a pair of elasticized leg openings.

Another example of a training pant is that in U.S. Pat. No. 4,940,464, which is assigned to the assignee of the present invention. This training pant is different from that described in U.S. Pat. No. 4,646,362 in that it has stretchable side panels that maintain the central absorbent assembly snugly against the child's body.

Besides children, adults experience incontinence problems and some adults use several types of garments to hide or provide for their incontinence. For example, adults who experience incontinence generally try to hide their problem by use of feminine pads, diapers, or larger diaper-like articles that are sized to fit the adult anatomy. These adults have need of a satisfactory three-dimensional garment to care for their needs. Also, women's menstrual needs need to be provided for with improved pant-like garments that can be discreetly worn with different types of clothing.

It is therefore an object of the present invention to provide an improved disposable three-dimensional garment, such as a training pant or a garment to hide adult incontinence. This object is solved by the disposable three dimensional garment of independent claim 1. Further advantageous features, aspects and details of the invention are evident from the dependent claims, the description and the drawings. The claims are intended to be understood as a first non-limiting approach of defining the invention in general terms.

The invention provides a child's disposable training pant having stretch gradient side panels for improved ease of use and improved fit and performance and method of making the same.

In one form of the invention, there is provided a disposable three-dimensional absorbent garment comprising a full outer cover including an inner surface, an outer surface, a pair of side sections, and waist and leg openings. A pair of stretchable side members are provided on the inner surface of the outer cover at respective side sections to provide stretch to the outer cover. An absorbent medium is provided at the crotch section of the outer cover.

Visual characters may be provided on the outer cover. A pair of stretchable side members are operatively joined with the inner surface at respective side sections, and each stretchable side member includes a stretch gradient between the waist opening and a leg opening.

Another form of the present invention provides a disposable three-dimensional absorbent garment comprising a full outer cover having an inner surface, an outer surface, a pair of side sections, and waist and leg openings. A pair of triangularly-shaped stretchable side members are joined with the inner surface at the side sections. A stretchable waistband is joined to the waist opening. A vertex of each stretchable side member is positioned generally adjacent the waistband. An absorbent medium is provided at the crotch section of the outer cover.

The above-mentioned and other features and objects of this invention and the manner of attaining them will become more apparent, and the invention itself will be better understood by reference to the following description of the examples of the invention, taken in conjunction with the accompanying drawings, wherein:
Fig. 1 is a perspective view of a preferred embodiment of the present invention;
Fig. 2 is a perspective view of a modification of the embodiment in Fig. 1 without visual characters;
Fig. 3 is a perspective view of another modification of the embodiment in Fig. 1 without visual characters;
Fig. 4 is a top plan view of the embodiment of Fig. 1 with the seams unjoined and the embodiment laid flat with the liner partially broken away to expose interior elements; Fig. 5 is a view similar to Fig. 4 of the modification in Fig. 2;
Fig. 6 is a broken-away view similar to Fig. 4 of the modification in Fig. 3;
Fig. 7 is a cross-sectional view illustrating one arrangement of various elements;
Fig. 8 is a top plan view illustrating one process of attaching stretchable members; and
Fig. 9 is similar to Fig. 8 with the stretchable members in a relaxed, joined state.

### Definitions

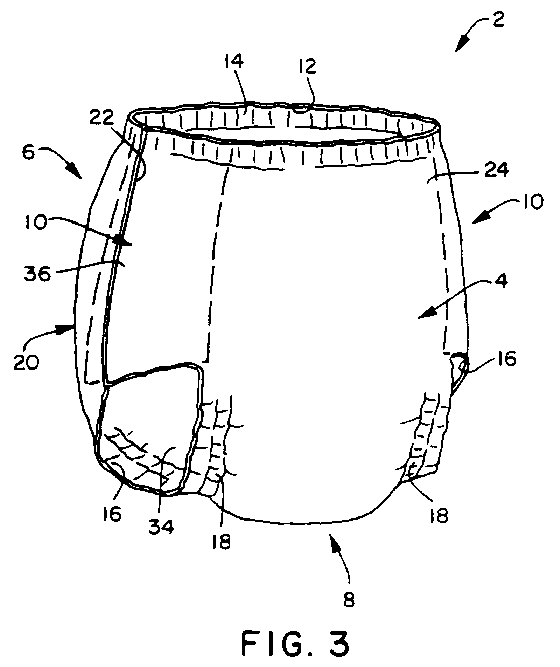

Within the context of this specification, each term or phrase below will include the following meaning or meanings:
(a) "SBL" means a stretch bonded laminate that is at least a two-layered composite in which one layer is a gatherable layer and the other layer a stretchable layer. The layers are joined together when the stretchable layer is in a stretched condition so that upon relaxing the layers, the gatherable layer is gathered.
(b) "Two-dimensional" refers to a garment that can be opened without tearing any structure and laid in a flat condition. These garments, such as diapers, do not have continuous leg and waist openings, and require a fastening device, such as tapes, to attach about the wearer.
(c) "Three-dimensional" refers to a garment similar to shorts or pants in that they have continuous leg and waist openings that are bounded by material of which the garment is made.
(d) "Full outer cover" means that the outer cover is one piece or unitary and defines the form of a three-dimensional pant.
(e) "Horizontal" or "horizontally" refers to a left-to-right and/or right-to-left direction as viewed in the Figures, particularly with reference to Fig. 3, and does not require an orientation congruent with the true horizontal. An element that is horizontally disposed or oriented includes that element oriented in the true horizontal or within about 45 degrees of true horizontal.
(f) "Member" when used in the singular can have the dual meaning of a single panel-like member, or a plurality of bands or strings.
(g) "Operatively joined" with reference to the attachment of a stretchable member to another element means that the stretchable member when attached to or connected to or treated with heat with the element makes that element stretchable. The joining can be either directly, such as joining the stretchable member directly on the element, or can be joined indirectly by means of a second element disposed between the stretchable member and the first element.
(h) "Triangularly shaped" includes not only a true triangle, but also a truncated triangle. The sides may be linear or curvilinear. The shape is three-sided.
(i) "Stretch", "stretchability", "stretch characteristics", and variations thereof can be used interchangeably and mean that the material which is being described can be stretched and upon relaxing will tend to resume its original shape. The force necessary to stretch the material is the tension of or on the stretched material.

These definitions are not intended to be limiting, and these terms may be defined with additional language in the remaining portion of the specification.

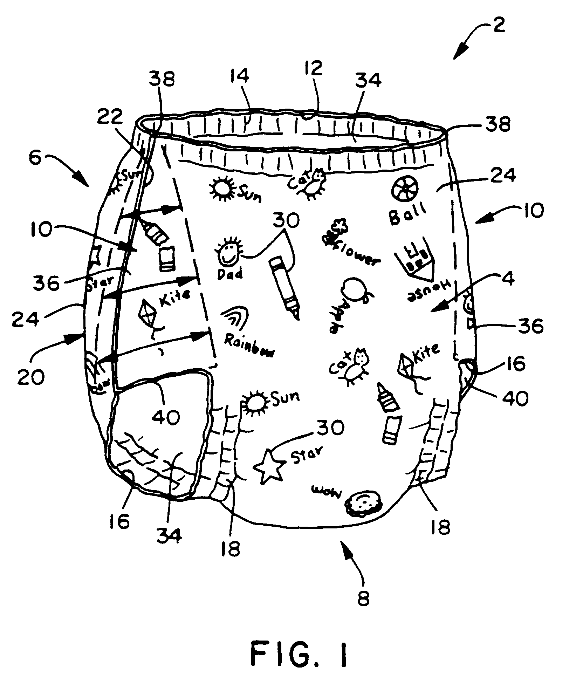

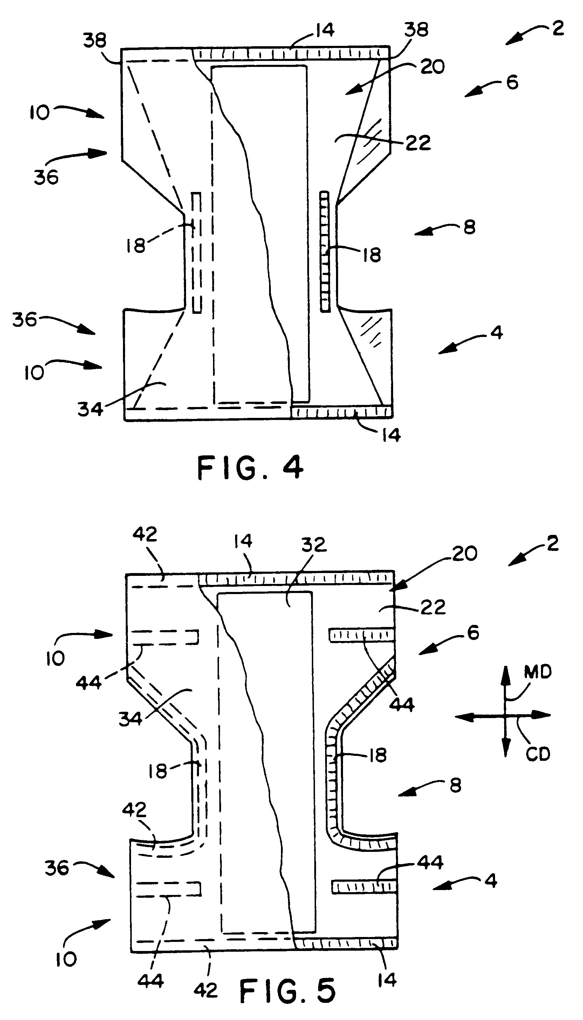

Although the current invention is contemplated as being used by adults or by children as a child's training pant, the following description will focus solely on a child's training pant for ease in describing and understanding the invention. Referring primarily to Figs. 1 and 4, disposable three-dimensional training pant 2 comprises in major part front section 4, back section 6, crotch section 8, a pair of side sections 10, waist opening 12 having a stretchable waistband 14, and a pair of leg openings 16 having respective stretchable leg bands 18.

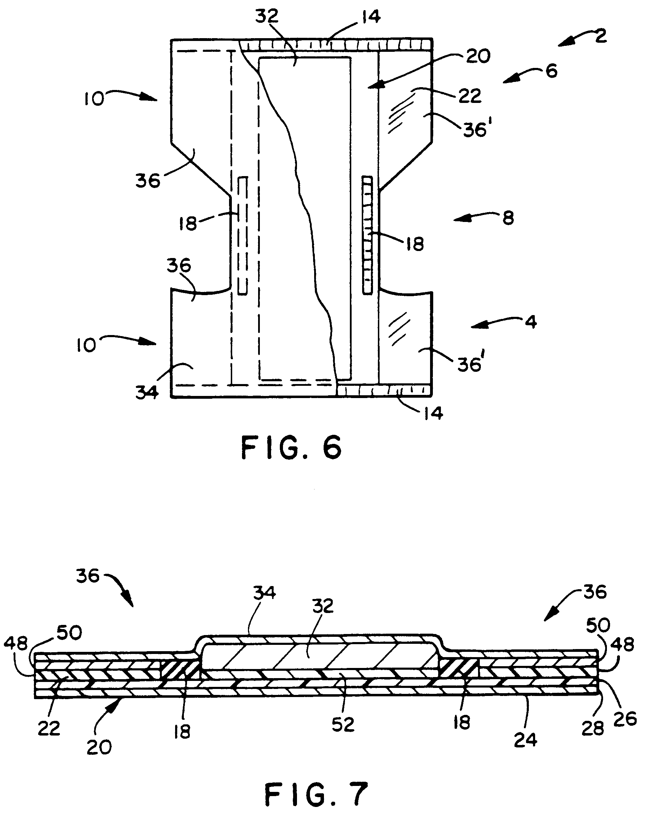

Referring also to Fig. 7 along with Figs. 1 and 4, training pant 2 further comprises a full outer cover 20 having an inner surface 22 and an outer surface 24. Outer cover 20 is preferably liquid impermeable, and comprises an inner layer 26 of a liquid impermeable material joined to an outer layer 28 of nonwoven material. This type of two-layered outer cover is more specifically described in U.S. Pat. No. 4,646,362. When outer cover 20 is a two-ply laminate with inner layer 26 and outer layer 28, then inner surface 22 is that surface of inner layer 26 that faces inwardly toward the wearer, and outer surface 24 is that surface of outer layer 28 that faces outwardly away from the wearer. If desired, outer cover 20 can be liquid impermeable and vapor permeable.

Outer cover 20 also includes a plurality of visual characters 30 on its outer surface 24. These characters 30 are not necessary for the waste containment performance of the invention and may be eliminated if desired. These characters 30 can include any type or number and combination of animals, letters, objects, toys, scenes, and the like which may be of interest to and/or educational for the child. If outer cover 20 is a single ply outer cover, then visual characters 30 are printed, or in any other suitable manner formed, on outer surface 24. visual characters 30 can be on inner layer 26 such that they are sandwiched between inner layer 26 and outer layer 28, or can be on the surface of outer layer 28 that is opposite from inner layer 26.

Training pant 2 further comprises absorbent medium 32 fixed between outer cover 20 and body side liner 34 in any suitable manner known in the art.

Training pant 2 further includes a pair of stretchable side members 36 operatively joined with inner surface 22 of outer cover 20 to make side sections 10 stretchable. By being operatively joined to inner surface 22, all of outer surface 24 of outer cover 20 is totally or fully exposed to view, thereby providing a more pant-like appearance. The pant-like appearance is further enhanced by visual characters 30 being included at side sections 10, as well as front section 4, back section 6, and crotch section 8.

Continuing to refer to Figs. 1 and 4, each stretchable side member 36 includes a vertex 38 that is generally adjacent waist opening 12 and a base 40 generally adjacent a respective leg opening 16. Stretchable side members 36 are preferably disposed between outer cover 20 and liner 34, and preferably attached to both outer cover 20 and liner 34. As illustrated in Fig. 1, it can be appreciated that the triangularly-shaped design of stretchable side members 36 provides a stretch-gradient to respective side sections 10, in which the amount of stretchability increases from waist opening 12 to a respective leg opening 16 as illustrated by the horizontal arrows within stretchable side member 36 of Fig. 1. Within the context of this description, as a stretchable material is increasingly stretched, the force applied by that material to or against or upon the wearer also increases. By providing this type of stretch gradient to side sections 10, training pant 2 of the present invention has the preferred characteristics of being easily pulled up the legs to the waist by the wearer, while providing a snug fit against the wearer in order to maintain absorbent medium 32 snugly in place to absorb waste material. Because stretchability is provided about the total periphery of waist opening 12 by waistband 14, it is easier to open or expand as compared to a waist opening that is only partially stretchable. In addition, absorbent medium 32 is maintained snugly in place by leg bands 18 and stretchable side members 36, thereby providing the desired waste containment.

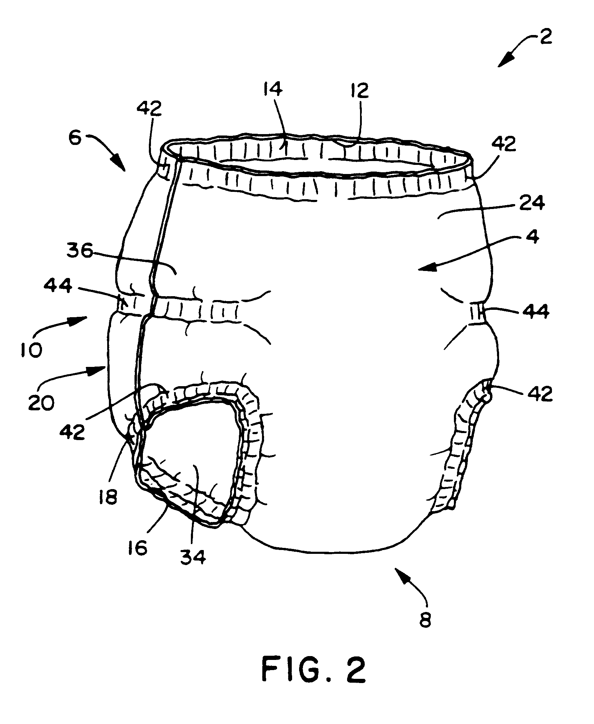

Referring now to Figs. 2 and 5, training pant 2 has been modified by substituting the generally triangularly-shaped stretchable side member 36 in each side section 10 by a plurality of generally horizontally disposed stretchable bands 42, 44. Fig. 2 illustrates these stretchable bands 42, 44 in side section 10 as including that portion of stretchable waistband 14 in side section 10, that portion of stretchable leg band 18 in side section 10, and an intermediate stretchable band 44. Naturally, a fewer number or greater number of bands 42, 44 can be provided, and may also take the shape of strings or slender thread-like filaments. The stretch gradient can be provided in several ways. For example, the stretchable materials of which waistband 14, intermediate stretchable band 44, and stretchable leg band 18 are made can vary in stretchability or cross-sectional area such that the stretchability of each side section 10 increases from the waist opening 12 to a respective leg opening 16.

A stretch gradient can also be provided for each stretchable side band 42, 44 in Fig. 2 by using the same type of stretchable material and selectively treating the material. For example, stretchable leg band 18 would not receive any selective treatment, whereas intermediate stretchable band 44 could receive a first amount of selective treatment, and that portion of stretchable waistband 14 in side section 10 could receive a second selective treatment greater than the first treatment of band 44, in which the treatments minimize or decrease the stretchability. A stretch gradient can also be provided by using different types of stretchable materials. For example, waistband 14 would be made of a material having one stretchability, intermediate stretchable band 44 could be made of another material having a second stretchability greater than waistband 14, and leg band 18 could be made of a material having a third stretchability greater than intermediate stretchable band 14, thereby providing increased stretchability from the waist opening 12 to a respective leg opening 16.
As illustrated in Fig. 5, waistband 14, intermediate band 44, and leg bands 18 are preferably disposed between outer cover 20 and liner 34, and preferably attached to both outer cover 20 and liner 34.

Referring now to Figs. 3 and 6, another modification to training pant 2 illustrated in Fig. 1 has generally rectangularly-shaped side members 36'. In order to provide the desired stretch gradient in which the stretchability increases in a direction from waist opening 12 to leg openings 16, selected portions of each rectangularly-shaped stretchable side member 36' can be selectively treated or modified. For example, a plurality of bond points, which would not cause holes through each side member 36', could be provided in increasing concentration or density in a direction from a respective leg opening 16 to waist opening 12, thereby providing a stretch gradient in which the stretchability increases in a direction from waist opening 12 to a respective leg opening 16. Alternatively, a plurality of slits or openings (not shown) may also be provided through each side member 36' in increasing concentration or density in a direction from waist opening 12 to a respective leg opening 16.

The present invention contemplates other structures and designs that result in a stretch gradient between the waist opening 12 and respective leg openings 16, and the above descriptions are only representative. Although the above description has described the stretch gradient as having stretchability that increases in a direction from waist opening 12 to a respective leg opening 16, the present invention also contemplates the reverse, if desired, in which the stretchability would decrease from waist opening 12 to a respective leg opening 16. The stretch gradient also can increase-decrease-increase or decrease-increase-decrease between waist opening 12 and a respective leg opening 16. In other words, an intermediate portion of the stretch gradient can have greater or lesser stretchability than end portions thereof.

With reference to Fig. 7, stretchable side member 36 can be a two-layered composite. As described above, this composite can be made with several different processes and materials and configurations. One composite and process includes a heat-shrinkable layer 48 combined or associated in any suitable manner with any suitable nonwoven layer 50. This nonwoven layer 50 is preferably a polyester powder-bonded carded web. The composite can then be cut into any suitable shape for forming stretchable side members 36, for example into a triangular shape, rectangular shape, or bands or strings. Thereafter, stretchable side member 36 is placed on outer cover 20 such that layer 48 is contacting inner surface 22 of a side section 10. Layer 48 can then be thermally bonded to inner layer 22 and liner 34, thereby causing layer 48 to be stretchable. Suitable heat-shrinkable materials can be purchased from the Exxon Chemical Company.

The invention includes a 2-layer outer cover 20 in which the inner layer, such as liquid impermeable inner layer 26, is a heat-shrinkable material. Side sections 10 are made stretchable by application of heat as required for the heat-shrinkable material used.

Another process includes forming a composite into a desired shape for use as a stretchable side member 36. This composite can be a 2-layered composite in which one layer is a meltblown elastomer having a basis weight between about 40 to about 150 grams per square meter (gsm) and which is formed onto a second layer of a nonwoven material having a basis weight between about 5 to about 30 gsm. This formed composite can then be cut into any desired shape. Each formed and cut composite is then bonded, such as by thermal bonding, to inner surface 22 of outer cover 20 with the elastomer being sandwiched by the outer cover and the second layer of nonwoven material. In this case, outer cover 20 can be a nonwoven material having a basis weight between about 20 to about 60 gsm. Outer cover 20 can then be passed through a series of cross-directional softening rolls that cause only the composite to become stretchable in the horizontal or cross-direction, thereby providing stretchable side members 36.

Another process includes attaching a stretch bonded laminate material while in a stretched condition to inner surface 22 of side sections 10 and liner 34.

A liquid impermeable layer 52 can be provided between absorbent 32 and outer cover 20. However, liquid impermeable layer 52 can be eliminated in the case in which outer cover 20 comprises liquid impermeable inner layer 26 and outer layer 28.

Generally, waistband 14 will be a heat-shrinkable material preferably joined between outer cover 20 and liner 34, and thereafter selectively heated. In the case of leg bands 18, they are generally applied in a stretched condition before being preferably joined between outer cover 20 and liner 34.

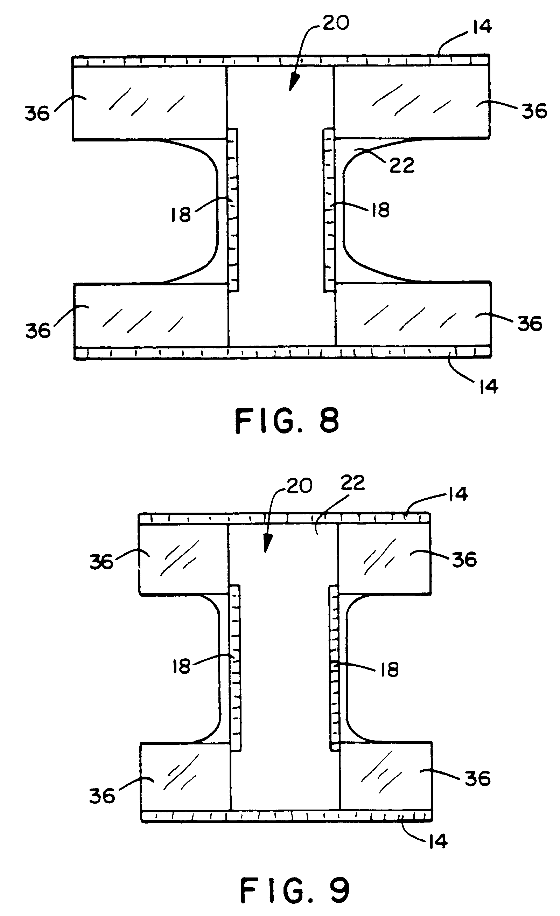

Referring now to Figs. 8 and 9, there is illustrated another method of joining stretchable side members 36 to outer cover 20. In this method, outer cover 20 and liner 34 are presized larger than the end-product size. The amount of presizing is naturally dependent upon the type of stretchable side member joined thereto. As illustrated in Fig. 8, stretchable waistband 14 and stretchable side members 36 are stretched in the cross-direction and then joined to inner surface 22 and liner 34 (not shown), while leg bands 18 are stretched in the machine direction and joined to inner surface 22 and liner 34 (not shown). Thereafter, waistband 14, stretchable side members 36, and leg bands 18 are allowed to relax, thereby gathering or shrinking outer cover 20 and liner 34 to the desired end-product size. Absorbent medium 32 is positioned between outer cover 20 and liner 34 during the placement of the stretchable members, and attached to outer cover 20 by lines of adhesive.

As earlier described, outer cover 20 can be a two-ply layer as described in U.S. Pat. No. 4,646,362.

Liner 34 may be a liquid permeable, hydrophilic or hydrophobic material, such as a spunbonded web composed of synthetic polymer filaments; a spunlace web; a spunbond-meltblown web; a meltblown web; or a bonded carded web composed of synthetic polymer fibers. Suitable synthetic polymers include polyethylene, polypropylene, polyester, and nylon.

Stretchable side members 36 can be made of any suitable materials, such as those of which the stretchable side members of U.S. Pat. No. 4,940,464 are made. Similarly, waistband 14 and leg bands 18 can be made of any suitable materials, such as those of which the waistband and leg bands of U.S. Pat. No. 4,940,464 are made.

Absorbent medium 32 may be made of any suitable absorbent material such as cellulosic fibers, synthetic fibers, absorbent gelling materials in the form of particles, fibers, layers, and the like. These various materials may be combined as mixtures or blends. They also may be discrete layers such as a discrete layer of cellulosic fiber and a discrete layer of absorbent gelling material, or a coform-type layer which is a blend or mixture of synthetic and cellulosic fibers formed as a coform layer with a discrete layer of absorbent gelling materials placed therewith. Suitable absorbent gelling materials can be inorganic materials such as silica gels or organic compounds such as cross-linked polymers. Examples include polyacrylamides, polyvinyl alcohol, polyacrylates, acrylonitrile grafted starch, acrylic acid grafted starch, and the like. Absorbent medium 32 can also include a tissue wrap to maintain the integrity thereof.

## Claims

1. A disposable absorbent three-dimensional garment (2), comprising:
a full outer cover (20) including an inner surface (22), an outer surface (24), a pair of side sections (10), a waist opening (12) and a pair of leg openings (16);
an absorbent medium (32) at a crotch section (8); and
a pair of stretchable side members (36, 36') provided on the inner surface (22) of the outer cover (20) at respective side sections (10) to provide stretch to the outer cover (20),
whereby said full outer cover (20) is a two-layered cover (20) comprising an inner layer (26) of heat-shrinkable material and an outer layer (28) of nonwoven material, and said inner layer (26) of heat-shrinkable material of said two-layered full outer cover (20) is heat-treated at least at respective portions thereof defining said pair of side sections (10), whereby said side sections (10) are made stretchable,
wherein said side sections (10) have respective stretch gradients.

2. The garment of claim 1 further comprising a stretchable waistband (14) joined to said waist opening (12).

3. The garment of one of the preceding claims further comprising visual characters on said outer cover (20).

4. The garment of claim 3 wherein said visual characters are between said inner layer (26) and said outer layer (28).

5. The garment of claim 3 wherein said visual characters are on an outer surface (24) of said outer cover (20).

## Patentansprüche

1. Saugfähiges, dreidimensionales Wegwerfbekleidungsstück (2) mit:
einer Vollaußenhülle (20) mit einer inneren Oberfläche (22), einer äußeren Oberfläche (24), einem Paar von Seitenabschnitten (10), einer Taillenöffnung (12) und einem Paar von Beinöffnungen (16);
einem saugfähigen Medium (32) an einem Schrittabschnitt (8); und
einem Paar dehnbarer Seitenelemente (36, 36') an der inneren Oberfläche (22) der Außenhülle (20) an entsprechenden Seitenabschnitten (10), um der Außenhülle (20) eine Dehnung zu verleihen
wobei es sich bei der Vollaußenhülle (20) um eine zweilagige Hülle (20) mit einer inneren Schicht (26) aus wärmeschrumpfbarem Material und einer äußeren Schicht (28) aus Vliesmaterial handelt, und die innere Schicht (26) aus wärmeschrumpfbarem Material der zweilagigen Vollaußenhülle (20) zumindest an entsprechenden Bereichen hiervon, welche das Paar von Seitenabschnitten (10) ausbilden, wärmebehandelt ist, wodurch den Seitenabschnitten (10) Dehnbarkeit verliehen ist,
wobei die Seitenabschnitte (10) jeweils Dehnungsgradienten aufweisen.

2. Bekleidungsstück gemäß Anspruch 1, das des weiteren einen mit der Taillenöffnung (12) verbundenen dehnbaren Taillenbund (14) aufweist.

3. Bekleidungsstück gemäß einem der vorhergehenden Ansprüche, das des weiteren sichtbare Darstellungen auf der Außenhülle (20) aufweist.

4. Bekleidungsstück gemäß Anspruch 3, bei dem sich die sichtbaren Darstellungen zwischen der inneren Schicht (26) und der äußeren Schicht (28) befinden.

5. Bekleidungsstück gemäß Anspruch 3, bei dem sich die sichtbaren Darstellungen auf einer äußeren Oberfläche (24) der Außenhülle (20) befinden.

## Revendications

1. Vêtement tridimensionnel absorbant jetable (2), comprenant:
une enveloppe extérieure totale (20) comprenant une surface intérieure (22), une surface extérieure (24), une paire de sections latérales (10), une ouverture de taille (12) et une paire d'ouvertures de jambes (16);
un milieu absorbant (32) au niveau d'une section d'entrejambe (8); et
une paire d'éléments latéraux extensibles (36, 36') prévus sur la surface intérieure (22) de l'enveloppe extérieure (20) au niveau des sections latérales respectives (10) pour donner de l'extensibilité à l'enveloppe extérieure (20)
dans lequel ladite enveloppe extérieure totale (20) est une enveloppe à deux couches (20) comprenant une couche intérieure (26) d'un matériau thermorétrécissable et une couche extérieure (28) d'un matériau non tissé, et ladite couche intérieure (26) de matériau thermorétrécissable de ladite enveloppe extérieure totale (20) à deux couches est traitée thermiquement au moins dans les portions respectives de celle-ci définissant ladite paire de sections latérales (10), grâce à quoi lesdites sections latérales (10) sont rendues extensibles, dans lequel lesdites sections latérales (10) comprenent un gradient d'extensibilité respective.

2. Vêtement selon la revendication 1, comprenant en outre une ceinture extensible (14) réunie à ladite ouverture de taille (12).

3. Vêtement selon l'une des revendications précédents, comprenant en outre des motifs sur ladite enveloppe extérieure (20).

4. Vêtement selon la revendication 3, dans lequel lesdits motifs sont situés entre ladite couche intérieure (26) et ladite couche extérieure (28).

5. Vêtement selon la revendication 3, dans lequel lesdits motifs sont situés sur une surface extérieure (24) de ladite enveloppe extérieure (20).
